# EUROPÄISCHE PATENTANMELDUNG

(11) **EP 2 123 159 A1**
(43) Veröffentlichungstag der Anmeldung: **25.11.2009**
(21) Anmeldenummer: 08156621.8
(22) Anmeldetag: 21.05.2008
(51) Int. Cl.: A01N 47/18, A01N 47/22, A01N 43/80, C07D 275/04, C07D 275/06

(54) **(1,2-Benzisothiazol-3-yl)(thio)carbamate und (1,2-Benzisothiazol-3-yl)(thio)oxamate und deren Oxidationsformen als Pestizide**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung beinhaltet ein Verfahren zur Bekämpfung von tierischen Schädlingen, wobei (l,2-Benzisothiazol-3-yl)(thio)carbamate und (1,2-Benzisothiazol-3-yl)(thio)oxamate und deren Oxidationsformen der Formel (I) 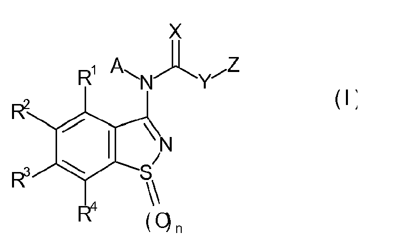 in welcher A, R¹, R², R³, R⁴, n, X, Y und Z die in der Beschreibung angegebenen Bedeutungen haben- als Pestizide Anwendung finden. Die Erfindung beinhaltet weiterhin spezifische Komponenten der Formel (I), sowie mehrere Verfahren zu ihrer Herstellung.

## Beschreibung

Die vorliegende Erfindung betrifft ein Verfahren zur Bekämpfung tierischer Schädlinge durch Anwendung insektizider und/oder akarizider Wirkstoffe insbesondere in Kombination mit Mitteln zur Wirkungssteigerung, sowie neue Schädlingsbekämpfungsmittel und mehrere Verfahren zu ihrer Herstellung.

In der Literatur sind bereits folgende Verbindungen beschrieben:

Substituierte 2-Cyano-benzolsulfonamide und die isomeren 3-Amino-1,2-benzisothiazol-1,1-dioxide sind beschrieben in der EP 33984. Dabei wird auch eine blattlausabtötende Wirksamkeit beschrieben. 3-Amino-1,2-benzisothiazole und deren Verwendung als Insektizide sind beschrieben in der EP 138762. In der EP 133418 sind 3-Acylamino-1,2-benzisothiazole als Pestizide, in der EP 110829 sind 3-Acylamino-1,2-benzisothiazol-1,1-dioxide als Insektizide und Akarizide in der EP 191734, EP 207891, und in der JP 1989/01319467 sind 3-Acylamino-1,2-benzisothiazol-1,1-dioxide als Pestizide; und in der DE 3544436 sind 3-(Diacylamino)-1,2-benzisothiazol-1,1-dioxide als Fungizide und Insektizide beschrieben. Weitere Derivate der 3-Amino-1,2-benzisothiazol-1,1-dioxide und deren isomerer Formen sind in der EP 86748, JP 1990/006496, WO 2007/057407 und WO 2007/113119 als Insektizide beansprucht. In der WO 2003/087072 wird die Herstellung von 1,2-Benzisothiazolen, in der DE 3112164 wird die Herstellung von 1,2-Benzisothiazolyl-oxamaten und deren Verwendung als Arzneimittel beschrieben. Über eine Verwendung als Schädlingsbekämpfungsmittel wird nichts berichtet. Eine antimikrobielle Wirkung der Verbindung Ethyl-(7-nitro-1,2-benzisothiazol-3-yl)carbamat wird erwähnt bei P. Vicini, Farmaca 44, 511-517 (1989).

In der Literatur wurde bereits ebenfalls beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat und Phosphinothricin beschrieben (US 6 645 914, EP-A2 0 036 106). Der Einsatz von Ammoniumsulfat als Formulierhilfs-mittel ist für bestimmte Wirkstoffe und Anwendungen ebenfalls beschrieben (WO 92/16108), es dient dort aber zur Stabilisierung der Formulierung, nicht zur Wirkungssteigerung.Desweiteren werden Kombinationen von Ammoniumsalzen mit insektiziden Wirkstoffen beschrieben in WO 07/068356, WO 07/068428, WO 07/068355, WO 07/068357, und WO 07/068350. Auf diese Veröffentlichungen wird hiermit ausdrücklich Bezug genommen.

Die gemäß den oben genannten Schriften bereits bekannten Wirkstoffe weisen bei ihrer Anwendung Nachteile auf, sei es,
(a) dass sie keine oder aber eine nur unzureichende insektizide und/oder akarizide Wirkung gegen tierische Schädlinge besitzen oder
(b) dass nur ein zu geringes Spektrum an Schadtieren bekämpft werden kann.

Aufgabe der vorliegenden Erfindung ist es daher, entsprechende Benzoisothiazol-Derivate bereitzustellen, welche als Insektizide und/oder Akarizide, insbesondere mit einer zufrieden stellenden insektiziden und/oder akariziden Wirkung gegen tierische Schädlinge, mit einem breiten Spektrum gegenüber tierischen Schädlingen, mit einer hohen Selektivität und guten Verträglichkeit in Nutzpflanzenkulturen, sowie welche in Kombination mit weiteren Mitteln zur Verbesserung der Wirksamkeit insbesondere gegen schwierig zu bekämpfende Insekten und Spinnmilben, eingesetzt werden können.

Es wurden nun Benzothiazol-Derivate der allgemeinen Formel (I) gefunden, 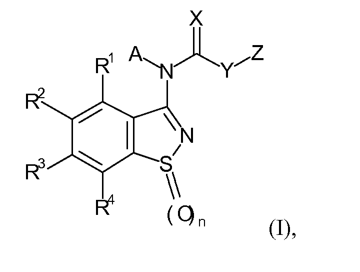 wobei
R¹, R², R³ und R⁴ unabhängig voneinander für
Wasserstoff, Halogen, Carbamoyl, Thiocarbamoyl, Nitro, Cyano, Hydroxy, SF₅, C₁-C₆-Alkyl, C₂-C₆-Alkenyl, C₂-C₆-Alkinyl, C₃-C₆-Cycloalkyl, C₃-C₆-Cycloalkyl-C₁-C₄-Alkyl, C₁-C₄-Halogenalkyl, C₁-C₄-Halogenalkylthio, C₁-C₄-Alkylthio, C₁-C₄-Alkylsulfmyl, C₁-C₄-Halogenalkylsulfinyl, C₁-C₄-Alkylsulfonyl, C₁-C₄-Halogenalkylsulfonyl, C₁-C₄-Alkylcarbonyl, C₁-C₆-Alkoxy, C₁-C₆-Alkoxycarbonyl, C₃-C₆-Alkenyloxy, C₃-C₆-Cycloalkyloxy, C₃-C₆-Cycloalkyloxy-C₁-C₄-Alkyl, C₁-C₄-Halogenalkoxy, Cₗ-C₄-Alkoxy-C₁-C₄-Alkyl, Aryloxy, Hetaryloxy, Aryl-C₁-C₄-Alkyloxy, Hetaryl-C₁-C₄-Alkyloxy, (C₁-C₄-Alkoxy)carbonyl, O-Acetyl, (C₁-C₄-Alkyl)amino, Di-(C₁-C₄-alkyl)amino, C₃-C₆-Trialkylsilyl, Aryl, Hetaryl, Aryl-C₁-C₆-Alkyl oder Hetaryl-C₁-C₆-Alkyl stehen,
   wobei diese Reste unsubstituiert sein können oder einen, zwei oder mehrere Reste aus der Gruppe
Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl und Hetaryl tragen können;
A für Wasserstoff, (C₁-C₆-Alkyl)carbonyl, (C₁- C₆-Alkoxy)carbonyl, (C₁-C₆-Alkylthio)carbonyl, (C₁-C₆-Alkyl)thiocarbonyl, (C₁-C₆- Alkoxy)thiocarbonyl, (C₁-C₆-Alkylthio)thiocarbonyl, C₁-C₈-Alkyl, C₃-C₆-Cycloalkyl, C₂-C₆- Alkenyl, C₁-C₄-Alkoxy, C₁-C₄-Alkylthio, C₁-C₄-Alkylsulfinyl, C₁-C₄-Alkylsulfonyl, C₁-C₄-Halogenalkyl, Aryl, Hetaryl, C₁-C₆-Arylalkyl oder C₁-C₆-Hetarylalkyl steht,
   wobei diese Reste unsubstituiert sein können oder einen, zwei oder mehrere Reste aus der Gruppe
Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl und Hetaryl tragen können;
   oder
   - A: für den Rest 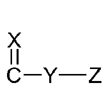 steht,
   - n: für 0, 1 oder 2 steht,
   - X: für O oder S steht,
   - Y: für O, S, oder C(=O) steht,
   - Z: für C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Aryl, Hetaryl, C₁-C₆-Arylalkyl, C₁-C₆-Hetarylalkyl, und für den Fall, dass Y für C(=O) steht, auch für C₁-C₈-Alkoxy, C₁-C₄-Haloalkoxy, Aryloxy oder Hetaryloxy steht,
   wobei Aryl und Hetaryl gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können,
sowie Salze von Verbindungen der Formel (I), deren Anwendung zur Bekämpfung von tierischen Schädlingen und die Wirkungssteigerung der Verbindungen der Formel (I) durch den Zusatz von Ammonium- oder Phosphoniumsalzen zur Anwendungslösung oder durch den Einbau dieser Salze in eine Formulierung enthaltend Verbindungen der Formel (I).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die (1,2-Benzisothiazol-3-yl)(thio)carbamate und (1,2-Benzisothiazol-3-yl)(thio)oxamate und deren Oxidationsformen sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.

Als bevorzugt, besonders bevorzugt und ganz besonders bevorzugt gelten Verbindungen der Formel (I), sowei ein Vefahren zur Bekämpfung von Schädlingen unter Anwendung der Verbindungen der Formel (I), wobei

R¹, R², R³ und R⁴ unabhängig voneinander bevorzugt für

Wasserstoff, Halogen, Cyano, Nitro, C₁-C₆-Alkyl, C₂-C₆-Alkenyl, C₂-C₆-Alkinyl, C₃-C₆-Cycloalkyl, C₃-C₆-Cycloalkyl-C₁-C₄-Alkyl, C₁-C₄-Halogenalkyl, C₁-C₄-Alkylthio, C₁-C₄-Alkylsulfonyl, C₁-C₄-Alkylcarbonyl, C₁-C₆-Alkoxy, C₁-C₆-Alkoxycarbonyl, C₃-C₆-Alkenyloxy, C₃-C₆-Cycloalkyloxy, C₃-C₆-Cycloalkyloxy-C₁-C₄-Allcyl, C₁-C₄-Halogenalkoxy, C₁-C₄-Alkoxy-C₁-C₄-Alkyl, Aryloxy, Hetaryloxy, Aryl-C₁-C₄-Alkyloxy, Hetaryl-C₁-C₄-Alkyloxy, (C₁-C₄-Alkoxy)carbonyl, O-Acetyl, Aryl, Hetaryl, Aryl-C₁-C₆-Alkyl oder Hetaryl-C₁-C₆-Alkyl stehen,
wobei diese Reste unsubstituiert sein können oder einen, zwei oder mehrere Reste aus der Gruppe

Halogen, Cyano, C₁-C₆-Alkyl, C₃-C₈-Cycloalkyl, C₁-C₄-Alkoxy tragen können,
R¹, R², R³ und R⁴ unabhängig voneinander besonders bevorzugt für
Wasserstoff, Halogen, Nitro, C₁-C₃-Alkyl, C₁-C₃-Halogenalkyl, C₁-C₃-Alkylthio, C₁-C₃-Alkylsulfonyl, C₃-C₆-Alkenyloxy, C₁-C₃-Alkoxy, C₁-C₂-Halogenalkoxy, C₁-C₄-Alkoxy-C₁-C₄-Alkyl, Aryloxy, Hetaryloxy, C₁-C₂-Arylalkyloxy, C₁-C₂-Hetarylalkyloxy, C₁-C₄-Alkoxycarbonyl, O-Acetyl stehen,
wobei diese Reste unsubstituiert sein können oder einen, zwei oder mehrere Reste aus der Gruppe
Halogen, C₁-C₄-Alkoxy tragen können,
R¹, R², R³ und R⁴ unabhängig voneinander ganz besonders bevorzugt für
Wasserstoff, Nitro, Methoxy, Ethoxy, Ethoxymethyl, Benzyloxy, Fluor, Chlor, Methyl, Ethyl, Trifluormethyl, OCHF₂, OCF₃, OCClF₂, OCH₂CH₂-OCH₃, SCH₃, SO₂CH₃, -OCH₂-CH=CH₂, -OCH₂-Phenyl stehen,
- A: bevorzugt für Wasserstoff, , (C₁-C₆-Alkyl)carbonyl, (C₁-C₆-Alkoxy)carbonyl, (C₁-C₆-Alkylthio)carbonyl, (C₁-C₆-Alkyl)thiocarbonyl, (C₁-C₆-Alkoxy)thiocarbonyl, (C₁-C₆-Alkylthio)thiocarbonyl, C₁-C₆-Alkyl, C₃-C₆-Cycloalkyl, C₂-C₆-Alkenyl, C₁-C₄-Alkoxy, C₁-C₄-Halogenalkyl, Aryl, Hetaryl, C₁-C₂-Arylalkyl oder C₁-C₂-Hetarylalkyl steht,
oder

- A: bevorzugt für den Rest 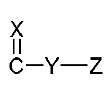 steht,
- A: besonders bevorzugt für Wasserstoff, (C₁-C₃-Alkyl)carbonyl, (C₁-C₃-Alkoxy)carbonyl, C₁-C₂-Alkyl, C₁-C₄-Alkoxy, C₁-C₄-Halogenalkyl, C₁-C₄-Halogenalkoxy, C₁-C₂-Arylalkyl oder C₁-C₂-Hetarylalkyl steht,
oder

- A: besonders bevorzugt für den Rest 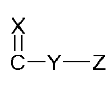 steht,
- A: ganz besonders bevorzugt für Wasserstoff, Acetyl, C₁-C₂-Alkyl, C₁-C₄-Alkoxy steht, oder

- A: ganz besonders bevorzugt für den Rest 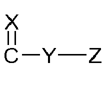 steht,
- n: bevorzugt steht für 0, 1 oder 2,
- n: besonders bevorzugt steht für 0 oder 2,
- n: weiterhin besonders bevorzugt steht für 2,
- X: bevorzugt und besonders bevorzugt steht für O oder S,
- X: ganz besonders bevorzugt steht für O,
- Y: bevorzugt steht für O, S, oder C(=O),
- Y: besonders bevorzugt für O oder S steht,
- Y: ganz besonders bevorzugt für O steht,
- Z: bevorzugt für C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Aryl, Hetaryl, C₁-C₆-Arylalkyl, C₁-C₆-Hetarylalkyl, und für den Fall, dass Y für C(=O) steht, auch für C₁-C₈-Alkoxy, C₁-C₄-Haloalkoxy, Aryloxy, Hetaryloxy steht,
- Z: besonders bevorzugt für C₁-C₄-Alkyl, C₁-C₄-Haloalkyl, C₂-C₆-Haloalkinyl, Aryl und für den Fall, dass Y für C(=O) steht, auch für C₁-C₄-Alkoxy steht,
- Z: ganz besonders bevorzugt für Methyl, Ethyl, Propyl, Isopropyl, tert. Butyl, Phenyl und für den Fall, dass Y für C(=O) steht, auch für Methoxy oder Ethoxy steht.

In einer weiteren Ausführungsform der Erfindung steht A nicht für Phenyl, falls n= 2 ist; bevorzugt stehen A, R¹, R², R³, R⁴ für Wasserstoff, falls n=2 ist. Ebenfalls in einer weiteren Ausführungsform ist R¹ verschieden von Wasserstoff, falls n=0 ist.

Die oben angegebenen einzelnen allgemeinen, bevorzugten, besonders bevorzugten und ganz besonders bevorzugten Bedeutungen für die Substituenten R¹ bis R⁴, n, X, Y, Z und A können beliebig miteinander kombiniert werden. Das heißt, dass Verbindungen der allgemeinen Formel (I) von der vorliegenden Erfindung umfasst sind, in welchen beispielswiese der Substituent R¹ eine bevorzugte Bedeutung aufweist und die Substituenten X und Y die allgemeine Bedeutung aufweisen oder aber beispielsweise der Substituent R² eine bevorzugte Bedeutung aufweist, der Substituent R³ eine besonders bevorzugte Bedeutung, der und die übrigen Substituenten die allgemeine Bedeutung aufweisen.

Gegenstand der vorliegenden Erfindung sind vorzugsweise auch die Lithium-, Natrium-, Kalium-, Magnesium-, Calcium-, Ammonium-, C₁-C₄-Alkyl-ammonium, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium, C₅- oder C₆-Cycloalkyl-ammonium-, Di-(C₁-C₂-alkyl)-benzyl-ammonium und Tri-(C₁-C₂-alkyl)-benzyl-ammonium-Salze von Verbindungen der Formel (I), in welcher R¹ bis R⁴, n, X, Y, Z und A die oben allgemeinen, bevorzugten, besonders bevorzugten und insbesondere bevorzugten Bedeutungen aufweisen und die nach allgemein üblichen Verfahren hergestellt werden können.

In der allgemeinen Formel (I) und allen übrigen Formeln in der vorliegenden Erfindung können die Reste Alkyl, Alkoxy, Haloalkyl, Alkoxyalkyl, Alkenyl, Alkinyl, Haloalkoxy, Alkylamino, Dialkylamino, Alkylthio und Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, Propyle wie n- oder i-Propyl, Butyle wie n-, iso- oder tert.-Butyl, Pentyle wie n-Pentyl, iso-Pentyl oder neo-Pentyl, Hexyle wie n-Hexyl, i-Hexyl, 3-Methylpentyl, 2,2-Dimethylbutyl oder 2,3-Dimethylbutyl, Heptyle, wie n-Heptyl, 1-Methylhexy oder 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Alkyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl. Der Begriff "Halo" wird erfindungsgemäß synonym zu "Halogen" verwendet.

Halogen bedeutet Fluor, Chlor, Brom oder lod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCI, CCI₃, CHCI₂, CH₂CH₂CI; Haloalkoxy ist z.B. OCF₃, OCHF₂, OCH₂F, CF₃CF₂O, OCH₂CF₃ und OCH₂CH₂CI; entsprechendes gilt für Haloalkenyl und andere durch Halogen substituierte Reste.

Die vorliegenden Verbindungen der allgemeinen Formel (I) können gegebenenfalls ein chirales Kohlenstoffatom aufweisen. Entsprechende chirale Kohlenstoffatome können insbesondere in dem Substituenten A und 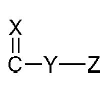 aufttreten

Gemäß den Regeln nach Cahn, Ingold und Prelog (CIP-Regeln) können diese Substituenten sowohl eine (R)- als auch eine (S)-Konfiguration aufweisen.

Von der vorliegenden Erfindung werden Verbindungen der allgemeinen Formel (I) sowohl mit (S)-als auch mit (R)-Konfiguration an den jeweiligen chiralen Kohlenstoffatomen umfasst, d.h., dass die vorliegende Erfindung die Verbindungen der allgemeinen Formel (I) erfasst, in welchen die betreffenden Kohlenstoffatome jeweils unabhängig voneinander
(1) eine (R)-Konfiguration; oder
(2) eine (S)-Konfiguration
   aufweisen. Wenn mehrere Chiralitätszentren in den Verbindungen der allgemeinen Formel (I) vorliegen, sind beliebige Kombinationen der Konfigurationen der Chiralitätszentren möglich, d.h. dass

(1) ein Chiralitätszentrum die (R)-Konfiguration und das andere Chiralitätszentrum die (S)-Konfiguration;
(2) ein Chiralitätszentrum die (R)-Konfiguration und das andere Chiralitätszentrum die (R)-Konfiguration; und
(3) ein Chiralitätszentrum die (S)-Konfiguration und das andere Chiralitätszentrum die (S)-Konfiguration
   aufweist.

Erfindungsgemäß umfasst sind daher insbesondere die folgenden Verbindungen der Formeln (I-1), (I-2) und (I-3)
(I-1) RS -Verbindung
(I-2) RR-Verbindung
(I-3) SS-Verbindung

Weiterer Gegenstand der vorliegenden Erfindung sind Verfahren zur Herstellung entsprechender Verbindungen der allgemeinen Formel (I) und/oder deren Salze.

In einer **ersten** Ausführungsform (A) der vorliegenden Erfindung erfolgt die Herstellung der Verbindungen der allgemeinen Formel (I) dadurch, dass man 3-Amino-1,2-benzoisothiazole der allgemeinen Formel (II) 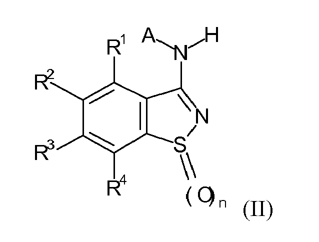 mit einem Säurederivat der allgemeinen Formel (III) 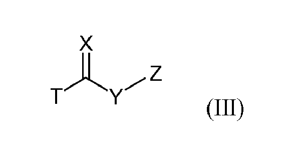 umsetzt, worin R¹ bis R⁴, n, X, Y, Z und A die vorstehende Bedeutung aufweisen
und T für Halogen und 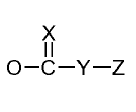 steht, bevorzugt für Chlor und

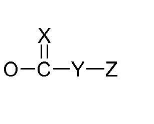

In einer **zweiten** Ausführungsform (B) der vorliegenden Erfindung erfolgt die Herstellung der Verbindungen der allgemeinen Formel (I) dadurch, dass man (Thio)carbamate und (Thio)oxamate der Formel (Ib) 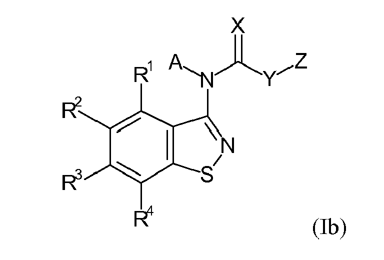 in welcher A, X, Y, Z, R¹, R², R³ und R⁴ die oben angegebenen Bedeutungen haben, mit einem Oxidationsmittel umsetzt.

In einer **dritten** Ausführungsform (C) der vorliegenden Erfindung erfolgt die Herstellung der Verbindungen der allgemeinen Formel (I) dadurch, dass man (Thio)carbamate und (Thio)oxamate der Formel (Ic) 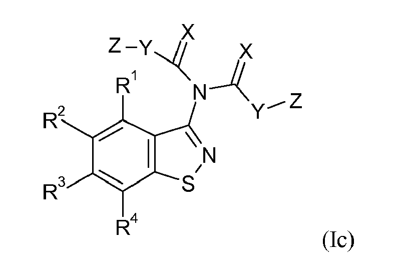 mit einem basischen oder sauren Spaltungsreagens unsetzt, worin A Wasserstoff bedeutet und R¹ bis R⁴, n, X, Y und Z die vorstehende Bedeutung aufweisen.

Alle dieser Verfahren führen zu erfindungsgemäßen Verbindungen der allgemeinen Formel (I).

In den jeweils vorstehend genannten Verfahrensvarianten werden jeweils inerte Lösemittel verwendet. Unter inerte Lösemittel werden im Sinne der vorliegenden Erfindung Lösemittel verstanden, die unter den jeweiligen Reaktionsbedingungen inert sind, d.h. insbesondere nicht mit den Edukten reagieren, jedoch nicht unter beliebigen Reaktionsbedingungen inert sein müssen.

Beispiele organischer Lösemittel, welche im Rahmen der vorliegenden Erfindung verwendet werden können, sind aromatische oder aliphatische Lösemittel, wie Benzol, Toluol, Xylol, Mesitylen, Hexan, Heptan, Octan, Cyclohexan,; aliphatische und aromatische Halogenkohlenwasserstoffe, wie Methylenchlorid, Dichlorethan, Chloroform, Kohlenstofftetrachlorid, Chlorbenzol, Dichlorbenzol, Ether, wie Diethylether, Dibutylether, Diisobutylether, Methyl-tert-butylether, Isopropylethylether, Diisopropylether, Tetrahydrofuran, und Dioxan; weiter auch Dimethylsulfoxid, und Säureamidderivate, wie N,N-Dimethylformamid, N,N-Dimethylacetamid und N-Methyl-2-pyrrolidon, sowie auch Carbonsäureester, wie Ethylacetat, oder aber auch Diglyme, Dimethylglycol; Nitrile wie Acetonitril, Propionitril oder Butyronitril sowie Ketone wie Aceton, Methylethyl-keton oder Cyclohexanon. Besonders bevorzugt sind Toluol, Xylol, Dichlorbenzol, Chlorbenzol, Acetonitril, Aceton, Butyronitril oder Ethylacetat. Allerdings ist die vorliegende Erfindung nicht auf die zuvor beispielhaft genannten Lösemittel beschränkt.

Die Reaktionstemperatur, bei welcher die Umsetzungen gemäß den vorstehenden Ausführungsformen durchgeführt werden können, kann in weiten Bereichen variieren. Entsprechende Temperaturen sind in den jeweiligen Ausführungsformen der Umsetzungen genannt. Darüber hinaus können die Umsetzungen bei einer Temperatur von 0 bis 100 °C, vorzugsweise 20 bis 70 °C, durchgeführt werden.

Der Umsetzungen der vorliegenden Erfindungen werden im Allgemeinen unter Normaldruck durchgeführt. Es ist jedoch auch möglich, unter erhöhtem Druck oder vermindertem Druck - im Allgemeinen zwischen 0,1 bar und 10 bar - zu arbeiten.

Die Verfahren zur Herstellung der erfindungsgemäßen Benzoisothiazole der allgemeinen Formel (I) werden gegebenenfalls in Gegenwart eines basischen oder sauren Reaktionshilfsmittels oder Spaltungsreagens durchgeführt.

Als solche kommen alle üblichen anorganischen oder organischen Basen infrage. Hierzu gehören beispielsweise Alkalimetall- oder Erdalkalimetallhydride, -hydroxide,-amide, -alkoholate, -acetate, -carbonate oder -hydrogencarbonate, wie beispielsweise Lithium-, Natrium-, Kalium- oder Calciumhydrid, Lithium-, Natrium- oder Kaliumamid, Natrium- oder Kaliummethylat, Natrium- oder Kaliumethylat, Natrium- oder Kaliumpropylat, Aluminiumisopropylat, Natrium- oder Kalium-tert.-butylat, Natrium- oder Kaliumhydroxid, Ammoniumhydroxid, Natrium-, Kalium- oder Calciumacetat, Ammoniumacetat, Natrium-, Kalium- oder Calciumcarbonat, Ammoniumcarbonat, Natrium- oder Kaliumhydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyldicyclohexylamin, N,N-Dimethylanilin, N,N-Dimethylbenzylamin, Pyridin, 2-Methyl-, 3-Methyl- und 4-Methylpyridin, 2,4-Dimethyl-, 2,6-Dimethyl-, 3,4-Dimethyl- und 3,5-Dimethylpyridin, 5-Ethyl-2-methyl-pyridin, N-Methylpyridin, 4-(N,N-Dimethylamino)-pyridin, Diazabicyclooctan (DABCO), Diazabicyclononen (DBN) oder Diazabicycloundecen (DBU).

Als saure Reaktionshilfsmittel kommen alle üblichen anorganischen oder organischen Säuren infrage. Hierzu gehören beispielsweise Mineralsäuren wie Schwefelsäure H₂SO₄, Phosphorsäure H₃PO₄ oder Salzsäure HCl oder organische Säuren wie Ameisensäure, Essigsäure oder Trifluoressigsäure.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii,

Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (IV) definiert 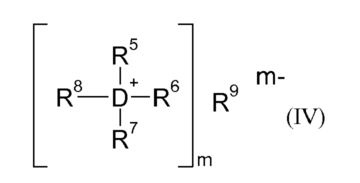 in welcher
- D: für Stickstoff oder Phosphor steht,
- D: bevorzugt für Stickstoff steht,
- R⁵, R⁶, R⁷, and R⁸: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,

- R⁵, R⁶, R⁷, and R⁸: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R⁵, R⁶, R⁷, and R⁸: besonders bevorzugt unabhängig voneinander für Wasserstoff, Methyl, Ethyl, n-Propyl, i-Propyl, n-Butyl, i-Butyl, s-Butyl oder t-Butyl stehen,
- R⁵, R⁶, R⁷, and R⁸: ganz besonders bevorzugt für Wasserstoff stehen,
- m: für 1, 2, 3 oder 4 steht,
- m: bevorzugt für 1 oder 2 steht,
- R⁹: für ein anorganisches oder organisches Anion steht,
- R⁹: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R⁹: besonders bevorzugt für Laktat, Sulfat, Monohydrogenphosphat, Dihydrogenphosphat, Nitrat, Thiosulfat, Thiocyanat, Citrat, Oxalat oder Formiat steht.
- R⁹: ganz besonders bevorzugt für Sulfat steht.

Die Ammonium- und Phosphoniumsalze der Formel (IV) können in einem breiten Konzentrationsbereich zur Steigerung der Wirkung von Pflanzenschutzmitteln enthaltend Verbindungen der Formle (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Selbst in diesen Fällen ist eine noch weiter gehende Wirkungssteigerung zu beobachten. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)ᵥ₋R' (V)

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- V: für Zahlen von 2 bis 30 steht.

Eine bevorzugte Gruppe von Penetrationsförderern sind Alkanolalkoxylate der Formel

R-O-(-EO-)ₙ-R' (V-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (V-b)

in welcher
- R: die oben angegebene Bedeutung hat,
- R': die oben angegebene Bedeutung hat,
- EO: für -CH₂-CH₂-O- steht,

- PO: für 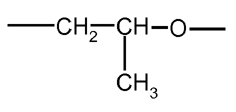 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (V-c)

in welcher
- R: die oben angegebene Bedeutung hat,
- R': die oben angegebene Bedeutung hat,
- EO: für -CH₂-CH₂-O- steht,
- PO: für 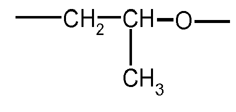 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (V-d)

in welcher
- R und: R' die oben angegebenen Bedeutungen haben,
- EO: für CH₂-CH₂-O- steht,
- BO: für 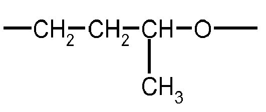 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (V-e)

in welcher
R und R^{'} die oben angegebenen Bedeutungen haben,
- BO: für 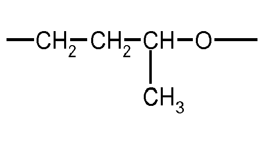 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (V-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (V-c) sei 2-Ethyl-hexyl-alkoxylat der Formel 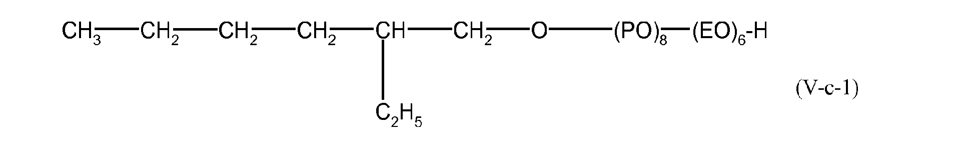 in welcher
- EO: für -CH₂-CH₂-O- steht,
- PO: für 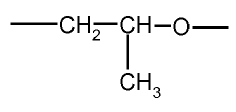 steht und
die Zahlen 8 und 6 Durchschnittswerte darstellen, genannt.

Als Beispiel für ein Alkanol-Alkoxylat der Formel (V-d) sei die Formel

CH₃-(CH₂)₁₀-O-(-EO-)₆-(-BO-)₂-CH₃ (V-d-1)

in welcher
- EO: für CH₂-CH₂-O- steht,
- BO: für 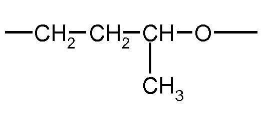 steht und
die Zahlen 10, 6 und 2 Durchschnittswerte darstellen, genannt.

Besonders bevorzugte Alkanol-Alkoxylate der Formel (V-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (V-f-1)

CH₃-(CH₂)ₜ-CH₂-O-(-CH₂-CH₂-O-)ᵤ-R' (V-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer sind in folgender Tabelle aufgeführt."Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

**Tabelle 1:**

| # | Wirkstoff | Salz | Penetrationsförderer |
|---|---|---|---|
| 1 | I, IA, IB | Ammoniumsulfat | Gemäß Test |
| 2 | I, IA, IB | Ammoniumlaktat | Gemäß Test |
| 3 | I, IA, IB | Ammoniumnitrat | Gemäß Test |
| 4 | I, IA, IB | Ammoniumthiosulfat | Gemäß Test |
| 5 | I, IA, IB | Ammoniumthiocyanat | Gemäß Test |
| 6 | I, IA, IB | Ammoniumcitrat | Gemäß Test |
| 7 | I, IA, IB | Ammoniumoxalat | Gemäß Test |
| 8 | I, IA, IB | Ammoniumformiat | Gemäß Test |
| 9 | I, IA, IB | Ammoniumhydrogenphosphat | Gemäß Test |
| 10 | I, IA, IB | Ammoniumdihydrogenphosphat | Gemäß Test |
| 11 | I, IA, IB | Ammoniumcarbonat | Gemäß Test |
| 12 | I, IA, IB | Ammoniumbenzoat | Gemäß Test |
| 13 | I, IA, IB | Ammoniumsulfit | Gemäß Test |
| 14 | I, IA, IB | Ammoniumbenzoat | Gemäß Test |
| 15 | I, IA, IB | Ammoniumhydrogenoxalat | Gemäß Test |
| 16 | I, IA, IB | Ammoniumhydrogencitrat | Gemäß Test |
| 17 | I, IA, IB | Ammoniumacetat | Gemäß Test |
| 18 | I, IA, IB | Tetramethylammoniumsulfat | Gemäß Test |
| 19 | I, IA, IB | Tetramethylammoniumlaktat | Gemäß Test |
| 20 | I, IA, IB | Tetramethylammoniumnitrat | Gemäß Test |
| 21 | I, IA, IB | Tetramethylammoniumthiosulfat | Gemäß Test |
| 22 | I, IA, IB | Tetramethylammoniumthiocyanat | Gemäß Test |
| 23 | I, IA, IB | Tetramethylammoniumcitrat | Gemäß Test |
| 24 | I, IA, IB | Tetramethylammoniumoxalat | Gemäß Test |
| 25 | I, IA, IB | Tetramethylammoniumformiat | Gemäß Test |
| 26 | I, IA, IB | Tetramethylammoniumhydrogenphosphat | Gemäß Test |
| 27 | I, IA, IB | Tetramethylammoniumdihydrogenphosphat | Gemäß Test |
| 28 | I, IA, IB | Tetraethylammoniumsulfat | Gemäß Test |
| 29 | I, IA, IB | Tetraethylammoniumlaktat | Gemäß Test |
| 30 | I, IA, IB | Tetraethylammoniumnitrat | Gemäß Test |
| 31 | I, IA, IB | Tetraethylammoniumthiosulfat | Gemäß Test |
| 32 | I, IA, IB | Tetraethylammoniumthiocyanat | Gemäß Test |
| 33 | I, IA, IB | Tetraethylammoniumcitrat | Gemäß Test |
| 34 | I, IA, IB | Tetraethylammoniumoxalat | Gemäß Test |
| 35 | I, IA, IB | Tetraethylammoniumformiat | Gemäß Test |
| 36 | I, IA, IB | Tetraethylammoniumhydrogenphosphat | Gemäß Test |
| 37 | I, IA, IB | Tetraethylammoniumdihydrogenphosphat | Gemäß Test |

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln,Semiochemicals oder auch mit Mitteln zur Verbesserung der Pflanzeneigenschaften vorliegen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren, Angießen und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

### Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken.

Verwendet man beispielsweise 4-Methoxy-1,2-benzisothiazol-3-amino-1,1-dioxid und Chlorameisensaeure-methylester als Ausgangsstoffe, so kann der Verlauf des erfindungsgemäßen Verfahrens der ersten Ausführungsform durch das folgende Formelschema veranschaulicht werden: 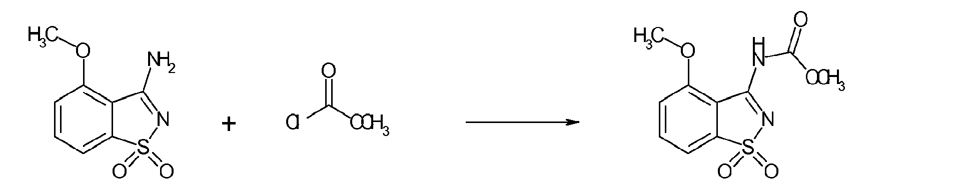 3-Amino-1,2-Benzisothiazole der Formel (II) sind bekannt, oder können nach allgemeinen Synthesemethoden hergestellt werden. Die EP°138762 beschreibt beispielsweise die Synthese von 3-Amino-4-chlor-1,2-benzisothiazol [CAS-RN 25380-80-7]. Die EP 33984 beschreibt die Synthese von 4-Chlor-3-amino-, 4-Fluor-3-amino- und 4-Methyl-3-amino-1,2-benzisothiazol-1,1-dioxid aus den entsprechenden 2-Cyano-benzolsulfonsäurechloriden. Die Herstellung aromatischer 2-Cyanobenzolsulfonsäurehalogenide ist in der DE 860051 beschrieben.

In der DE 3015113 werden 3-Amino-1,2-benzisothiazol-1,1-dioxide der allgemeinen Formel (II) als Zwischenprodukte beschrieben.

Die 3-Amino-1,2-benzisothiazole der Formel (II) sind auch teilweise kommerziell erhältlich, z.B. 3-Amino-1,2-benzisothiazol [CAS-RN 23031-78-9] oder das 3-Amino-5-nitro-benzisothiazol [CAS-RN 84387-89-3].

Ein allgemeines Verfahren zur Synthese von N-substituierten 3-Amino-1,2-benzisothiazolen der Formel (II) ist beschrieben in J. Org. Chem. 25, 413-16 (1960).

Die EP 105732 beschreibt N-substituierte 3-Amino-1,2-benzisothiazol-1,1-dioxide als Arzneimittel.

Verwendet man beispielsweise 4-Methyl-1,2-benzisothiazol-3-amino-1,1-dioxid und Di-tert-butyldicarbonat als Ausgangsstoffe, so kann der Verlauf des erfindungsgemäßen Verfahrens der ersten Ausführungsform auch durch das folgende Formelschema veranschaulicht werden:

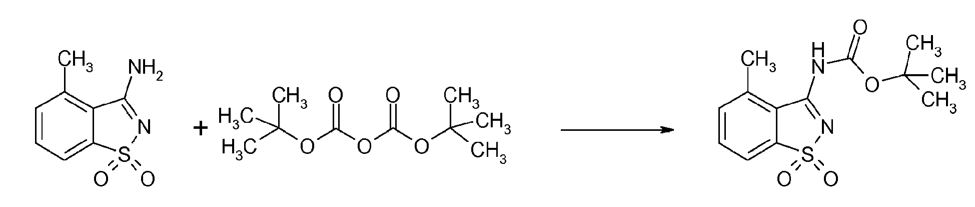

Verwendet man beispielsweise Dimethyl-(1,2-benzisothiazol-3-yl)imidodicarbonat und Wasserstoffperoxid als Ausgangsstoffe, so kann der Verlauf des erfindungsgemäßen Verfahrens der zweiten Ausführungsform durch das folgende Formelschema veranschaulicht werden:

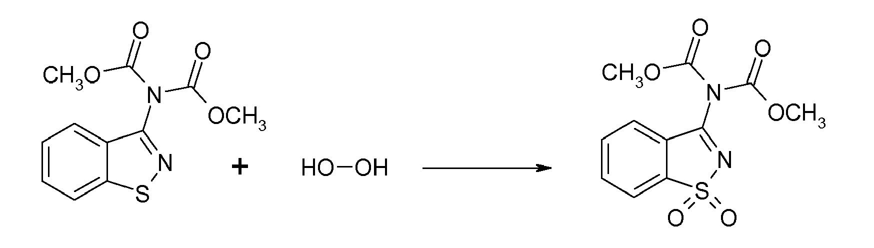

In der DE 3544436 wird die Oxidation der 3-Amino-1,2-benzisothiazole der allgemeinen Formel (II) mit 3-Chlor-perbenzoesäure beschrieben.

Verwendet man beispielsweise Dimethyl-(1,1-dioxido-1,2-benzisothiazol-3-yl)imidodicarbonat und ein basisches oder saures Spaltungsreagens, so kann der Verlauf des erfindungsgemäßen Verfahrens der dritten Ausführungsform durch das folgende Formelschema veranschaulicht werden:

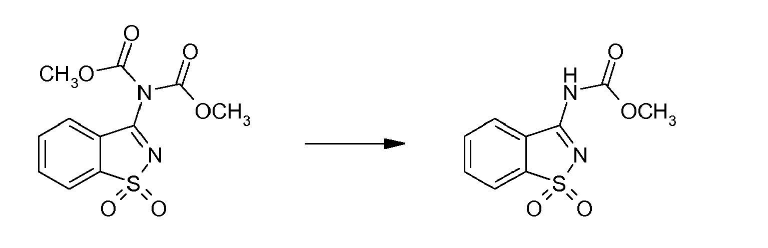

### Herstellung von Ausgangsverbindungen der Formel (II)

### 1. Herstellung von 4-(2-Methoxyethoxy)-1,2-benzisothiazol-3-amin-1,1-dioxid

### Stufe 1A : Herstellung von 2-Amino-6-(2-methoxyethoxy)-benzonitril

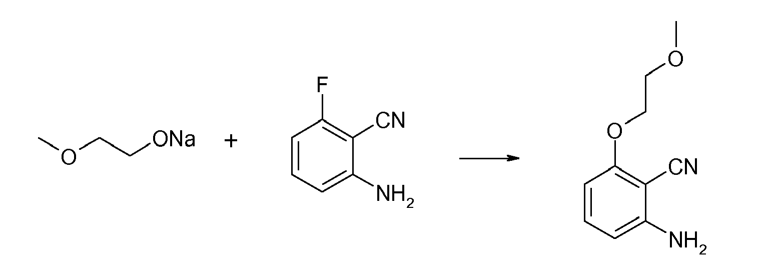

Zu einer Lösung von 3.2 g (23.5 mmol) 2-Amino-6-fluorbenzonitril in 10 ml trockenem DMF werden 25 g (51 mmol) einer 20 % igen Lösung von Natrium-2-methoxyethanolat in Methoxyethanol gegeben und 16 Stunden unter Rückfluss erhitzt. Nach dem Abkühlen wird mit 0.5 ml Wasser versetzt und im Vakuum eingeengt. Man nimmt in Essigsäureethylester und Wasser auf. Die wässrige Phase wird mit Essigsäureethylester nachextrahiert, die vereinigten organischen Phasen werden getrocknet und i. Vak. eingeengt. Es verbleiben 4 g Produkt mit einem Gehalt (NMR) > 95 %. Theoretische Ausbeute 84.1 %.
δ ([D₆]-DMSO) = 3.33 (s, 3H); 3.65-3.67 (t, 2H); 4.12-4.15 (t, 2H); 5.76 (br. s, 2H); 6.22-6.24 (m, 1H); 6.36-6.38 (m, 1H); 7.15-7.19 (m, 1H) ppm.

### Stufe 1B : Herstellung von 2-Amino-6-(2-methoxyethoxy)-benzolsulfonsäurechlorid

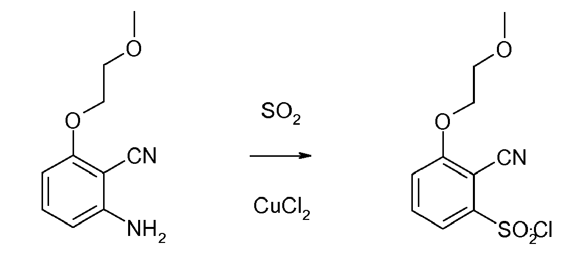

70 ml Essigsäure werden auf 10°C abgekühlt und unter Kühlung mit SO₂ gesättigt. Nach weiterem Abkühlen auf 0°C erfolgt die Zugabe von 1.4 g (8.2 mmol) Kupfer(II)chlorid-dihydrat, gelöst in 10 ml Wasser. Anschließend wird noch 15 Minuten nachgerührt. Danach wird zu dieser Lösung eine weitere Lösung [Herstellung: 4.0 g (20.8 mmol) 2-Amino-6-(2-methoxyethoxy)-benzonitril und 40 ml Essigsäure und 9 ml konzentrierte Salzsäure bei 0 bis 2°C vorlegen, 1.8 g (26.1 mmol) NaNO₂, gelöst in 10 ml Wasser, langsam zutropfen und weitere 30 Minuten bei 0°C nachrühren] bei 0°C schnell zugetropft. Nach langsamer Erwärmung auf Raumtemperatur wird 18 Stunden bei Raumtemperatur gerührt. Der Ansatz wird auf 500 ml Eiswasser gegeben und der entstandene hellbraune Feststoff wird abgesaugt und mit kaltem Wasser nachgewaschen. Es verbleiben 3.5 g Rohprodukt, das direkt weiter umgesetzt wird. Theoretische Ausbeute 61 %.

### Stufe 1C : Herstellung von 4-(2-Methoxyethoxy)-1,2-benzisothiazol-3-amin-1,1-dioxid

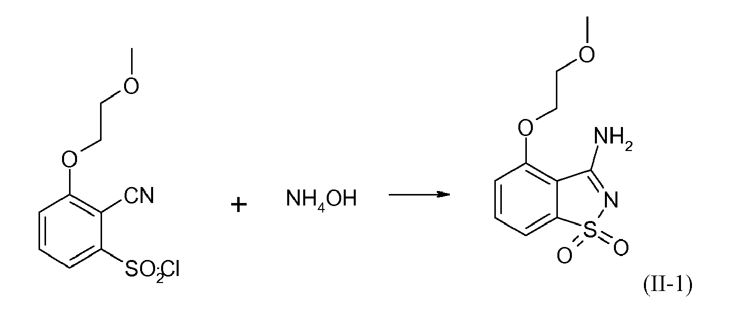

0.55 g (2 mmol) 2-Amino-6-(2-methoxyethoxy)-benzolsulfonsäurechlorid werden in 20 ml 25 % ige Ammoniak-Lösung eingetragen und zunächst 1 Stunde bei Raumtemperatur und dann noch 18 Stunden bei 45°C gerührt. Die wässrige Phase wird mit Dichlormethan versetzt, wobei ein beiger Feststoff ausfällt. Dieser wird abgesaugt und getrocknet. Man erhält 150 mg 4-(2-Methoxyethoxy)-1,2-benzisothiazol-3-amin-1,1-dioxid , Reinheitsgehalt (LC/MS) 93.9 %, Ausbeute: 29.4 % d. Theorie.
δ ([D₆]-DMSO) = 3.33 (s, 3H); 3.76-3.78 (t, 2H); 4.42-4.44 (m, 2H); 7.46-7.49 (m, 2H); 7.75-7.80 (m, 1H); 8.90 (br. s, 2H) ppm.

### 2. Herstellung von 4-( Prop-2-en-1-yloxy)-1,2-benzisothiazol-3-amin-1,1-dioxid

### Stufe 2A : Herstellung von 2-Amino-6-(prop-2-en-1-yloxy)-benzonitril

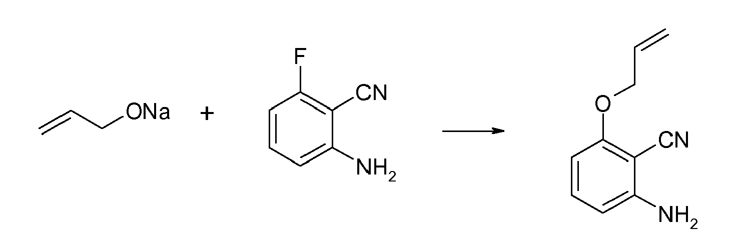

2.3 g Natrium-Späne (100 mmol) werden portionsweise zu 25.0 g (430 mmol) Allylalkohol gegeben und bis zur vollständigen Auflösung gerührt. Diese Lösung wird aus einem Tropftrichter zu einer Lösung von 7.5 g (55.1 mmol) 2-Amino-6-fluorbenzonitril in 20 ml trockenem DMF zugetropft und 16 Stunden unter Rückfluss erhitzt. Nach dem Abkühlen wird mit 0.5 ml Wasser versetzt und im Vakuum eingeengt. Man nimmt in Dichlormethan und Wasser auf. Die wässrige Phase wird mit Dichlormethan nachextrahiert, die vereinigten organischen Phasen werden über Magnesiumsulfat getrocknet und i. Vak. eingeengt. Es verbleiben 8.4 g Produkt mit einem Gehalt von 75 % (GC/MS) das direkt weiter umgesetzt wird. Theoretische Ausbeute 65.6 %.

### Stufe 2B : Herstellung von 2-Amino-6-(prop-2-en-1-yloxy)-benzolsulfonsäurechlorid

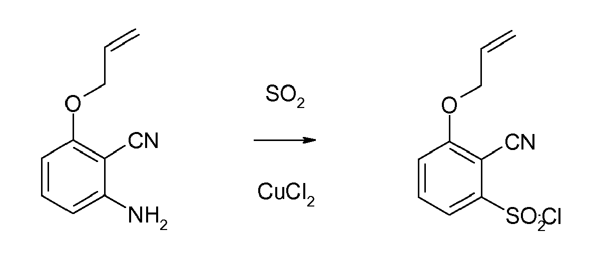

140 ml Essigsäure werden auf 10°C abgekühlt und unter Kühlung mit SO₂ gesättigt. Nach weiterem Abkühlen auf 0°C erfolgt die Zugabe von 3.32 g (19.5 mmol) Kupfer(II)chlorid-dihydrat, gelöst in 14 ml Wasser. Anschließend wird noch 15 Minuten nachgerührt. Danach wird zu dieser Lösung eine weitere Lösung [Herstellung: 8.9 g (51.1 mmol) 2-Amino-6-(prop-2-en-1-yloxy)-benzonitril und 65 ml Essigsäure und 20 ml konzentrierte Salzsäure bei 0 bis 2°C vorlegen, 4.15 g (60.1 mmol) NaNO₂, gelöst in 13 ml Wasser, langsam zutropfen und weitere 30 Minuten bei 0°C nachrühren] bei 0°C schnell zugetropft. Nach langsamer Erwärmung auf Raumtemperatur wird 18 Stunden bei Raumtemperatur gerührt. Der Ansatz wird auf 750 ml Eiswasser gegeben und der entstandene hellbraune Feststoff wird abgesaugt und mit kaltem Wasser nachgewaschen. Es verbleiben 5.5 g Rohprodukt, das direkt weiter umgesetzt wird. Theoretische Ausbeute 55.7 %.

### Stufe 2C : Herstellung von 4-(Prop-2-en-1-yloxy)-1,2-benzisothiazol-3-amin-1,1-dioxid

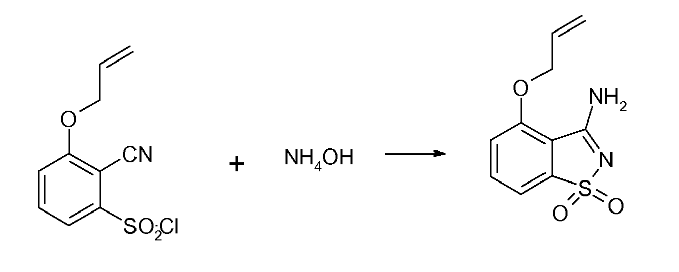

0.5 g (1.94 mmol) 2-Amino-6-(prop-2-en-1-yloxy)sulfonsäurechlorid werden in 40 ml 25 % ige Ammoniak-Lösung eingetragen und zunächst 2 Stunden bei 40°C und dann noch 18 Stunden bei Raumtemperatur und nochmals 1 Stunde bei 40°C gerührt. Die wässrige Phase wird mit DCM aufgerührt, wobei ein brauner Feststoff ausfällt. Dieser wird abgesaugt. Die wässrige Phase wird mit DCM nachextrahiert, die vereinigten organischen Phasen werden über MgSO4 getrocknet und i. Vak. eingeengt. Das Rohprodukt wird durch präparative HPLC gereinigt (Kromasil 100 C 18,Gradient mit Acetonitril [enthält 0.01 % Ameisensäure] / Wasser = 30 : 70. Es verbleiben 0.26 g Feststoff vom Reinheitsgehalt (LC/MS) 100 %, Ausbeute: 56.3 % d. Theorie.
δ ([D₆]-DMSO) = 4.90-4.92 (m, 2H); 5.31-5.34 (m, 1H); 5.39-5.44 (m, 1H); 6.07-6.16 (m, 1H); 7.40-7.45 (m, 2H); 7.57 (br. s, 1H); 7.73-7.77 (m, 1H); 8.97 (br. s, 1H) ppm.

### Beispiel 1

### Herstellung von Ethyl-[4-(2-methoxyethoxy)-1,1-dioxido-1,2-benzisothiazol-3-yl]carbamat (I-1)

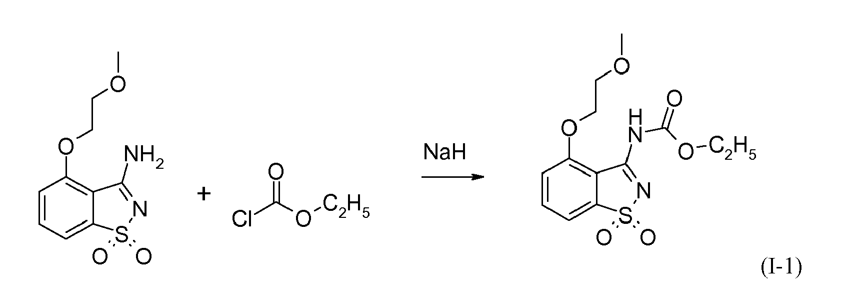

150 mg (0.59 mmol) 4-(2-Methoxyethoxy)-1,2-benzisothiazol-3-amin-1,1-dioxid werden in 35 ml absolutem Tetrahydrofuran vorgelegt und mit 60 mg (1.5 mmol) 60 % igem Natriumhydrid versetzt. Nach 2 Stunden bei 50 °C wird auf 20 °C abgekühlt. Unter Rühren wird eine Lösung aus 110 mg (1.01 mmol) Ethylchlorocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf. Die wässrige Phase wird mit DCM nachextrahiert, die vereinigten organischen Phasen werden über MgSO₄ getrocknet und i. Vak. eingeengt. Es verbleiben 80 mg Ethyl-[4-(2-methoxyethoxy)-1,1-dioxido-1,2-benzisothiazol-3-yl]carbamat (I-1), Gehalt (LC/MS): 89 %, Ausbeute: 37.1 % der Theorie.

### Beispiel 2

### Herstellung von Ethyl-(1,1-dioxido-1,2-benzisothiazol-3-yl)carbamat (I-2)

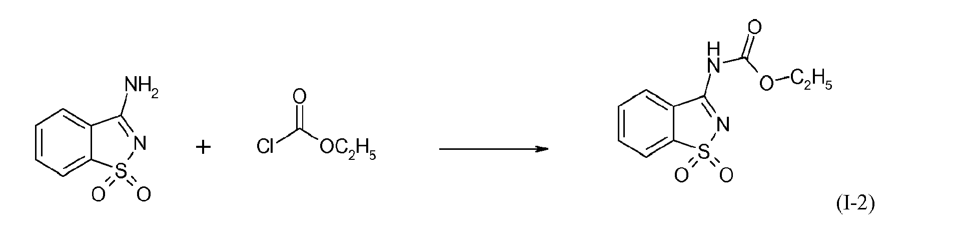

Man legt 300 mg (2.0 mmol) 1,2-Benzisothiazol-3-amin-1,1-dioxid in 25 ml trockenem Tetrahydrofuran vor und gibt portionsweise 130 mg (3.25 mmol) 60 % iges Natriumhydrid dazu. Es wird 2 h bei 50°C gerührt, auf Raumtemperatur abgekühlt und anschließend langsam 0.24 g (2 mmol) Chlorameisensäureethylester, gelöst in 5 ml trockenem Tetrahydrofuran, dazugegeben. Danach rührt man weitere 18h bei Rückfluss. Nach dem Abkühlen wird das Lösungsmittel i. Vak. entfernt. Das Rohprodukt wird durch präparative HPLC gereinigt (Kromasil 100 C 18,Gradient mit Acetonitril [enthält 0.01 % Ameisensäure] / Wasser = 40 : 60. Es verbleiben 0.06 g Ethyl-(1,1-dioxido-1,2-benzisothiazol-3-yl)carbamat (I-2) als weißer Feststoff vom Reinheitsgehalt (LC/MS) 100 %, Ausbeute: 14.3 % d. Theorie.

### Beispiel 3

### Methyl-(4-chlor-1,1-dioxido-1,2-benzisothiazol-3-yl)carbamat (I-3)

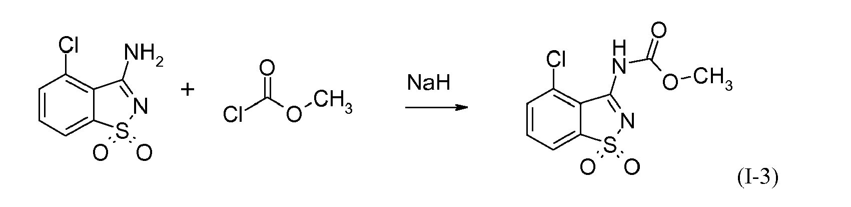

216.6 mg (1.0 mmol) 4-Chlor-1,2-benzisothiazol-3-amin-1,1-dioxid werden in 20 ml absolutem Tetrahydrofuran vorgelegt und mit 60 mg (1.5 mmol) 60 % igem Natriumhydrid versetzt. Nach 2 Stunden bei 50 °C wird auf 20 °C abgekühlt. Unter Rühren wird eine Lösung aus 103.9 mg (1.1 mmol) Methylchlorocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird mit Diisopropylether verrieben, der ausgefallene Niederschlag abgesaugt (270 mg). Nach Kieselgelchromatographie Dichlormethan /Methanol = 99.5:0.5) erhält man 170 mg Methyl-(4-chlor-1,1-dioxido-1,2-benzisothiazol-3-yl)carbamat vom Reinheitsgehalt (HPLC) 99.9 %, Ausbeute: 61.7 % d. Theorie.

### Beispiel 4

### 1-Methylethyl-(4-fluor-1,1-dioxido-1,2-benzisothiazol-3-yl)carbamat (I-4)

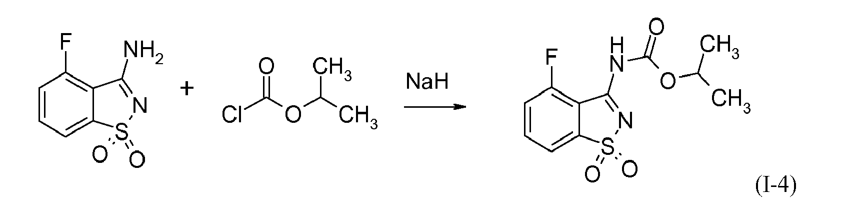

200.2 mg (1.0 mmol) 4-Fluor-1,2-benzisothiazol-3-amin-1,1-dioxid werden in 20 ml absolutem Tetrahydrofuran vorgelegt und mit 60 mg (1.5 mmol) Natriumhydrid versetzt. Nach 2 Stunden bei 50 °C wird auf 20 °C abgekühlt. Unter Rühren wird eine Lösung aus 134.8 mg (1.1 mmol) 1-Methylethylchlorocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird mit Diisopropylether verrieben, der ausgefallene Niederschlag abgesaugt (290 mg). Nach Kieselgelchromatographie (Dichlormethan/Methanol = 99.5:0.5) erhält man 190 mg 1-Methylethyl-(4-fluor-1,1-dioxido-1,2-benzisothiazol-3-yl)carbamat vom Reinheitsgehalt (HPLC) 97.6 %, Ausbeute: 64.8 % d. Theorie.

### Beispiel 5

### S-Methyl-(1,1-dioxido-1,2-benzisothiazol-3-yl)thiocarbamat (I-5)

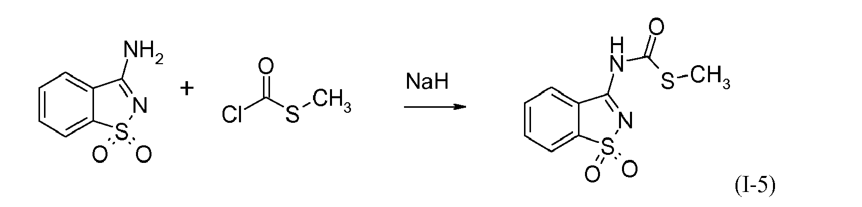

273.3 mg (1.5 mmol) 1,2-Benzisothiazol-3-amin-1,1-dioxid werden in 20 ml absolutem Tetrahydrofuran vorgelegt und mit 123 mg (3.07 mmol) Natriumhydrid versetzt. Nach 2 Stunden bei 50 °C wird auf 20 °C abgekühlt. Unter Rühren wird eine Lösung aus 248.8 mg (2.25 mmol) S-Methylchlorothiocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan und Wasser auf und säuert mit halbkonzentrierter Salzsäure an. Der ausgefallene Niederschlag wird abgesaugt und getrocknet. Man erhält 270 mg S-Methyl-(1,1-dioxido-1,2-benzisothiazol-3-yl)thiocarbamat vom Reinheitsgehalt (HPLC) 100 %, Ausbeute: 70.2 % d. Theorie.

### Beispiel 6

### Phenyl-(4-methoxy-1,1-dioxido-1,2-benzisothiazol-3-yl)carbamat (I-6)

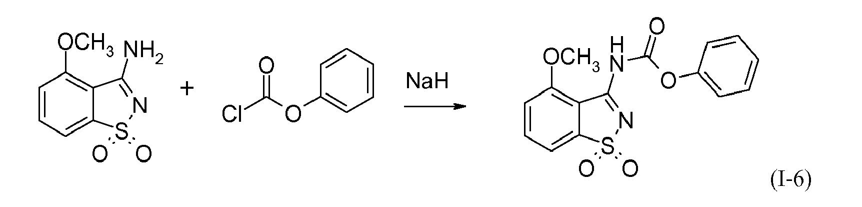

212.2 mg (1.0 mmol) 4-Methoxy-1,2-benzisothiazol-3-amin-1,1-dioxid werden in 12 ml absolutem Tetrahydrofuran vorgelegt und mit 82 mg (2.05 mmol) Natriumhydrid versetzt. Nach 2 Stunden bei 50 °C wird auf 20 °C abgekühlt. Unter Rühren wird eine Lösung aus 234.8 mg (1.5 mmol) Phenylchlorocarbonat in 2.5 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit Dichlormethan nachextrahiert, die vereinigten organischen Phasen werden getrocknet und i. Vak. eingeengt. Der Rückstand wird mit Cyclohexan/Essigsäureethylester = 3:1 an Kieselgel chromatographiert. Man erhält 60 mg Phenyl-(4-methoxy-1,1-dioxido-1,2-benzisothiazol-3-yl)carbamat vom Reinheitsgehalt (HPLC) 96.9 %, Ausbeute: 17.5 % d. Theorie.

### Beispiel 7

### Diphenyl-(4-methoxy-1,1-dioxido-1,2-benzisothiazol-3-yl)imidodicarbonat (I-7)

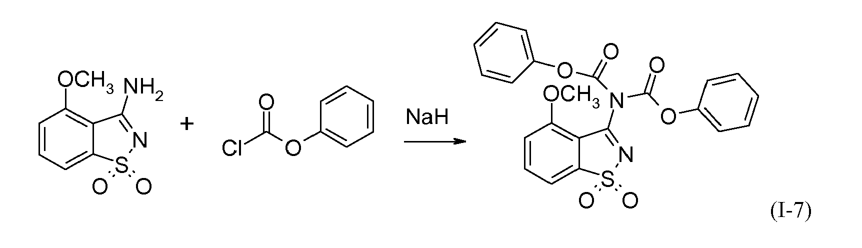

212.2 mg (1.0 mmol) 4-Methoxy-1,2-benzisothiazol-3-amin-1,1-dioxid werden in 12 ml absolutem Tetrahydrofuran vorgelegt und mit 82 mg (2.05 mmol) Natriumhydrid versetzt. Nach 2 Stunden bei 50 °C wird auf 20 °C abgekühlt. Unter Rühren wird eine Lösung aus 234.8 mg (1.5 mmol) Phenylchlorocarbonat in 2.5 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird mit Cyclohexan/Essigsäureethylester = 3:1 an Kieselgel chromatographiert. Man erhält 60 mg Diphenyl-(4-methoxy-1,1-dioxido-1,2-benzisothiazol-3-yl)imidodicarbonat vom Reinheitsgehalt (HPLC) 91.2 %, Ausbeute: 16.1 % d. Theorie.

### Beispiel 8

### Methyl (4-chlor-1,2-benzisothiazol-3-yl)carbamat (I-8)

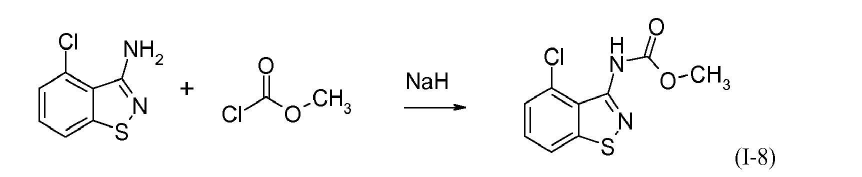

184.6 mg (1.0 mmol) 4-Chlor-1,2-benzisothiazol-3-amin werden in 20 ml absolutem Tetrahydrofuran vorgelegt und mit 60 mg (1.5 mmol) Natriumhydrid versetzt. Nach 2 Stunden bei 50 °C wird auf 20 °C abgekühlt. Unter Rühren wird eine Lösung aus 103.9 mg (1.1 mmol) Methylchlorocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird an Kieselgel chromatographiert, zuerst mit DCM/Methanol = 99.5:0.5, anschließend mit DCM/Essigsäureethylester = 40:1. Man erhält 70 mg Methyl (4-chlor-1,2-benzisothiazol-3-yl)carbamat vom Reinheitsgehalt (HPLC) 97.7 %, Ausbeute: 28.2 % d. Theorie.

### Beispiel 9

### S-Methyl-(4-chlor-1,2-benzisothiazol-3-yl)thiocarbamat (I-9)

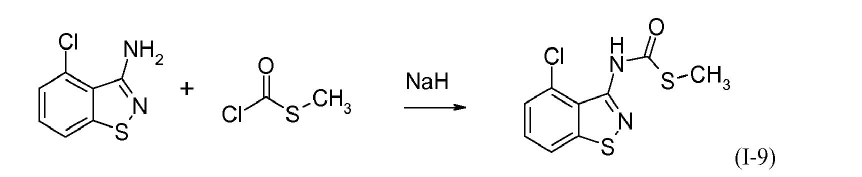

184.6 mg (1.0 mmol) 4-Chlor-1,2-benzisothiazol-3-amin werden in 20 ml absolutem Tetrahydrofuran vorgelegt und mit 60 mg (1.5 mmol) Natriumhydrid versetzt. Nach 2 Stunden bei 50 °C wird auf 20 °C abgekühlt. Unter Rühren wird eine Lösung aus 121.6 mg (1.1 mmol) S-Methylchlorothiocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird mit DCM an Kieselgel chromatographiert. Man erhält 70 mg S-Methyl-(4-chlor-1,2-benzisothiazol-3-yl)thiocarbamat vom Reinheitsgehalt (HPLC) 89.7 %, Ausbeute: 24.3 % d. Theorie.

### Beispiel 10

### Bis(1-methylethyl)-(4-chlor-1,2-benzisothiazol-3-yl)imidodicarbonat (I-10)

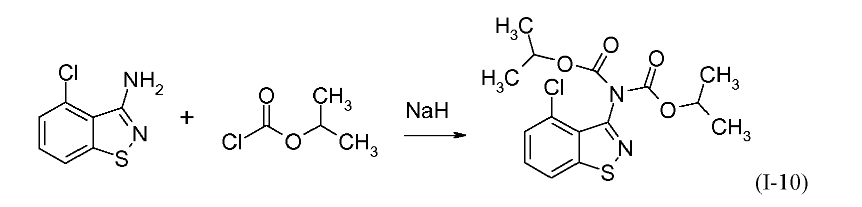

184.6 mg (1.0 mmol) 4-Chlor-1,2-benzisothiazol-3-amin werden in 20 ml absolutem Tetrahydrofuran vorgelegt und mit 60 mg (1.5 mmol) Natriumhydrid versetzt. Nach 2 Stunden bei 50 °C wird auf 20 °C abgekühlt. Unter Rühren wird eine Lösung aus 134.8 mg (1.1 mmol) 1-Methylethylchlorocarbonat in 3 ml absolutem Tetrahydrofuran zugetropft. Man erhitzt 16 Stunden unter Rückfluss, kühlt ab, versetzt mit 0.5 ml Wasser und engt im Vakuum ein. Man nimmt in Dichlormethan (DCM) und Wasser auf und säuert mit halbkonzentrierter Salzsäure an. Die wässrige Phase wird mit DCM nachextrahiert, die vereinigten organischen Phasen werden getrocknet und i. Vak. eingeengt. Der Rückstand wird an Kieselgel chromatographiert, erst DCM/Methanol = 99.5:0.5, anschließend Cyclohexan/Essigsäureethylester = 5:1. Man erhält 150 mg Bis(1-methylethyl)-(4-chlor-1,2-benzisothiazol-3-yl)imidodicarbonat vom Reinheitsgehalt (HPLC) 93.0 %, Ausbeute: 39.1 % d. Theorie.

### Beispiel 11

### Diethyl-(4-chlor-1,1-dioxido-1,2-benzisothiazol-3-yl)imidodicarbonat (I-11)

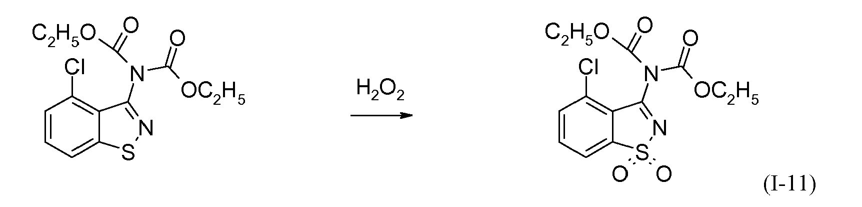

100 mg (0.304 mmol) Diethyl-(4-chlor-1,2-benzisothiazol-3-yl)imidodicarbonat werden zusammen mit 149 mg (3.242 mmol) Ameisensäure und 50 mg Ammoniummolybdat in 10 ml Dichlormethan (DCM) vorgelegt. Bei Raumtemperatur werden 443.3 mg (4.562 mmol) 35 %iges Wasserstoffperoxid zugetropft. Nach 4 Tagen Rühren wird auf Eiswasser gegossen, das überschüssige Peroxid durch Na-bisulfit-Lösung zerstört. Die organische Phase wird abgetrennt, getrocknet und i. Vak. eingeengt. Der Rückstand wird an Kieselgel mit Dichlormethan /Methanol = 99:1 chromatographiert. Man erhält 10 mg Diethyl-(4-chlor-1,1-dioxido-1,2-benzisothiazol-3-yl)imidodicarbonat vom Reinheitsgehalt (HPLC) 88.8 %, Ausbeute: 8.1 % d. Theorie.
Die in der Tabelle 1 beschriebenen Verbindungen der allgemeinen Formel (I) erhält man gemäß oder analog zu den oben beschriebenen Synthesebeispielen: 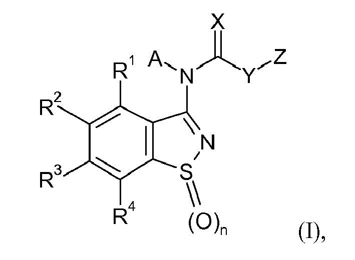 wobei die Reste R¹ bis R⁴, n, A, X,Y und Z die in der Tabelle 1 angegebenen Bedeutungen aufweisen

**Tabelle 1:**

| **Nr.** | **Phys. Daten** | **R¹** | **R²** | **R³** | **R⁴** | **n** | **A** | **X** | **Y** | **Z** |
|---|---|---|---|---|---|---|---|---|---|---|
| I-1 | ¹H-NMR | OCH₂CH₂-OCH₃ | H | H | H | 2 | H | O | O | C₂H₅ |
| I-2 | ¹H-NMR | H | H | H | H | 2 | H | O | O | C₂H₅ |
| I-3 | ¹H-NMR | Cl | H | H | H | 2 | H | O | O | CH₃ |
| I-4 | ¹H-NMR | F | H | H | H | 2 | H | O | O | C₃H₇-i |
| I-5 | ¹H-NMR | H | H | H | H | 2 | H | O | S | CH₃ |
| I-6 | ¹H-NMR | OCH₃ | H | H | H | 2 | H | O | O | C₆H₅ |
| I-7 | ¹H-NMR | OCH₃ | H | H | H | 2 | C(=O)-O- | O | O | C₆H₅ C₆H₅ |
| I-8 | ¹H-NMR | Cl | H | H | H | 0 | H | O | O | CH₃ |
| I-9 | ¹H-NMR | Cl | H | H | H | 0 | H | O | S | CH₃ |
| I-10 | ¹H NMR | Cl | H | H | H | 0 | C(=O)-OC₃H₇-i | O | O | C₃H₇-i |
| I-11 | ¹H-NMR | Cl | H | H | H | 2 | C(=O)-OC₂H₅ | O | O | C₂H₅ |
| I-12 | ¹H-NMR | OCH₃ | H | H | H | 2 | H | O | O | C₂H₅ |
| I-13 | ¹H-NMR | SCH₃ | H | H | H | 2 | H | O | O | C₂H₅ |
| I-14 | ¹H-NMR | SO₂CH₃ | H | H | H | 2 | H | O | O | C₂H₅ |
| I-15 | ¹H-NMR | -OCH₂-CH=CH₂ | H | H | H | 2 | H | O | O | C₂H₅ |
| I-16 | ¹H-NMR | CH₃ | H | H | H | 2 | H | O | O | CH₃ |
| I-17 | ¹H-NMR | OCH₃ | H | H | H | 2 | H | O | O | CH₃ |
| I-18 | ¹H-NMR | H | H | H | H | 2 | H | O | O | CH₃ |
| I-19 | ¹H-NMR | Cl | H | H | H | 0 | C(=O)-OC₂H₅ | O | O | C₂H₅ |
| I-20 | ¹H-NMR | Cl | H | H | H | 2 | H | O | O | C₃H₇-i |
| I-21 | ¹H-NMR | Cl | H | H | H | 2 | H | O | O | C₂H₅ |
| I-22 | ¹H-NMR | F | H | H | H | 2 | H | O | O | C₂H₅ |
| I-23 | ¹H-NMR | F | H | H | H | 2 | H | O | O | CH₃ |
| I-24 | ¹H-NMR | F | H | H | H | 2 | H | O | S | CH₃ |
| I-25 | ¹H-NMR | OCH₃ | H | H | H | 2 | H | O | S | CH₃ |
| I-26 | ¹H-NMR | Cl | H | H | H | 2 | H | O | S | CH₃ |
| I-27 | ¹H-NMR | Cl | H | H | H | 0 | C(=O)-OCH₃ | O | O | CH₃ |
| I-28 | ¹H-NMR | Cl | H | H | H | 0 | H | O | O | C₂H₅ |
| I-29 | ¹H-NMR | Cl | H | H | H | 0 | H | O | O | C₃H₇-i |
| I-30 | ¹H-NMR | Cl | H | H | H | 0 | H | O | CO | OC₂H₅ |
| I-31 | ¹H-NMR | H | H | H | CH₃ | 2 | H | O | O | CH₃ |
| I-32 | ¹H-NMR | H | H | H | CH₃ | 2 | H | O | O | C₂H₅ |
| I-33 | ¹H-NMR | H | H | H | CH₃ | 2 | H | O | S | CH₃ |
| I-34 | ¹H-NMR | OCH₂-C₆H₅ | H | H | H | 2 | H | O | O | C2H5 |
| I-35 | ¹H-NMR | H | N02 | H | H | 0 | H | O | CO | OC₂H₅ |

**I-1:**
   δ ([D₆]-DMSO) = 1.31 (t, 3H); 3.39 (s, 3H); 3.75-3.77 (m, 2H); 4.27-4.32 (q, 2H); 4.46-4.48 (m, 2H); 7.56-7.58 (m, 1H); 7.64-7.66 (m, 1H); 7.84-7.88 (m, 1H); 9.54 (br. s,1 H) ppm.
**I-2:**
   δ ([D₆]-DMSO) = 1.33 (t, 3H); 4.27-4.33 (q, 2H); 7.80-8.44 (m, 4H); 8.73 (br. s, 1H) ppm.
**I-3:**
   δ (CD₃CN) = 3.89 (s, 3H); 7.76-7.83 (m, 2H); 7.93 (dd, 1H); 9.1 (br. s, 1H) ppm.
**I-4:**
   δ (CD₃CN) = 1.35 (d, 6H); 5.08 (m, 1H); 7.49-7.54 (m, 1H); 7.79-7.80 (d, 1H); 7.85-7.90 (m, 1H); 8.21 (br. s, 1H) ppm.
**I-5:**
   δ ([D₆]-DMSO) = 2.38 (s, 3H); 7.83-7.90 (m, 2H); 8.06 (m, 1H); 8.43 (m, 1H) ppm.
**I-6:**
   δ (CD₃CN) = 4.13 (s, 3H); 7.26-7.36 (m, 3H); 7.42-7.49 (m, 3H); 7.55 (dd,1H); 7.84 (dd, 1H); 9.53 (br. s, 1H) ppm.
**I-7:**
   δ (CD₃CN) = 4.06 (s, 3H); 7.17-7.20 (m, 4H); 7.30-7.36 (m, 2H); 7.42-7.49 (m, 5H); 7.63 (dd, 1H); 7.90 (dd, 1H) ppm.
**I-8:**
   δ (CD₃CN) = 3.76 (s, 3H); 7.46-7.53 (m, 2H); 7.94 (dd, 1H); 8.2 (br. s, 1H) ppm.
**I-9:**
   δ ([D₆]-DMSO) = 2.30 (s, 3H); 7.57-7.63 (m, 2H); 8.21 (dd, 1H); 10.76 (s, 1H) ppm.
**I-10:**
   δ (CD₃CN) =1.10 (d, 6H); 1.13 (d, 6H); 4.99 (m, 2H); 7.51-7.58 (m, 2H); 8.01 (dd, 1H) ppm.
**I-11:**
   δ (CD₃CN) = 1.17 (t, 6H); 4.28 (q, 4H); 7.84 (d, 1H); 7.88 (t, 1H); 8.05 (d, 1H) ppm.
**I-12:**
   δ ([D₆]-DMSO) = 1.31 (t, 3 H); 4.09 (s, 3 H); 4.26-4.32 (q, 2 H); 7.53-7.55 (m, 1 H); 7.61-7.63 (m, 1 H); 7.85-7.89 (m, 1 H); 9.33 (s,1 H) ppm.
**I-13:**
   δ ([D₆]-DMSO) = 1.31 (t, 3 H); 2.68 (s, 3 H); 4.25-4.30 (q, 2 H); 7.77-7.94 (m, 4 H) ppm.
**I-14:**
   δ ([D₆]-DMSO) = 1.30 (t, 3 H); 3.60 (s, 3 H); 4.25-4.30 (q, 2 H); 8.11-8.17 (m, 1 H); 8.33-8.47 (m, 3 H) ppm.
**I-15:**
   δ ([D₆]-DMSO) = 1.16 (t, 3 H); 4.23-4.28 (q, 2 H); 4.80-4.82 (m, 2 H); 5.33-5.41 (m, 2 H); 5.97 (m, 1 H); 7.54-7.56 (m, 1 H); 7.74-7.76 (m, 1 H); 7.89-7.93 (m, 1 H) ppm.
**I-16:**
   δ ([D₆]-DMSO) = 2.59 (s, 3 H); 3.81 (s, 3 H); 7.68-7.82 (m, 3 H); 9.82 (br. s, 1 H) ppm.
**I-17:**
   δ ([D₆]-DMSO) = 3.84 (s, 3 H); 4.09 (s, 3 H); 7.53-7.56 (m,1 H); 7.62-7.64 (m, 1 H); 7.85-7.89 (m, 1 H); 9.38 (br. s, 1 H) ppm.
**I-18:**
   δ ([D₆]-DMSO) = 3.84 (s, 3 H); 7.82-7.90 (m, 2 H); 8.04-8.06 (m, 1 H); 8.42-8.44 (m, 1 H); 11.50 (br. s, 1 H) ppm.
**I-19:**
   δ (CD₃CN) = 1.10 (t, 6H); 4.19 (m, 4H); 7.51-7.59 (m, 2H); 8.01 (dd, 1H) ppm.
**I-20:**
   δ (CD₃CN) = 1.35 (d, 6H); 5.09 (m, 1H); 7.75-7.82 (m, 2H); 7.93 (dd, 1H); 9.04 (br. s, 1H) ppm.
**I-21:**
   δ (CD₃CN) = 1.35 (t, 3H); 4.34 (q, 2H); 7.75-7.83 (m, 2H); 7.93 (dd, 1H); 9.08 (br. s, 1H) ppm.
**I-22:**
   δ (CD₃CN) = 1.34 (t, 3H); 4.34 (q, 2H); 7.50-7.55 (m, 1H); 7.79-7.81 (m, 1H); 7.85-7.91 (m, 1H); 8.26 (br. s, 1H) ppm.
**I-23:**
   δ (CD₃CN) = 3.87 (s, 3H); 7.53-7.58 (m, 1H); 7.82-7.84 (m, 1H); 7.87-7.93 (m, 1H); 8.38 (br. s, 1H) ppm.
**I-24:**
   δ ([D₆]-DMSO) = 2.38 (s, 3H); 7.64-7.69 (m, 1H); 7.89-7.96 (m, 2H); 9.05 (br. s, 1H) ppm.
**I-25:**
   δ ([D₆]-DMSO) = 2.40 (s, 3H); 4.08 (s, 3H); 7.52 (d, 1H); 7.61 (d, 1H); 7.86 (dd, 1H); ∼ 10 (br., 1H) ppm.
**I-26:**
   δ ([D₆]-DMSO) = 2.39 (s, 3H); 7.82-7.88 (m, 2H); 8.02-8.06 (m, 1H); 9.25 (br., 1H) ppm.
**I-27:**
   δ (CD₃CN) = 3.71 (s, 6H); 7.55-7.62 (m, 2H); 8.04-8.06 (m, 1H) ppm.
**I-28:**
   δ ([D₆]-DMSO) = 1.86 (t, 3H); 4.80 (q, 2H); 8.07-8.14 (m, 2H); 8.55 (dd, 1H); 8.85 (br. s, 1H) ppm.
**I-29:**
   δ (CD₃CN) = 1.28 (d, 6H); 4.98 (m, 1H); 7.46-7.53 (m, 2H); 7.94 (dd, 1H); 8.11 (br. s, 1H) ppm.
**I-30:**
   δ (CD₃CN) = 1.38 (t, 3H); 4.40 (q, 2H); 7.51-7.57 (m, 2H); 7.98 (dd, 1H); 10.4 (br. s, 1H) ppm.
**I-31:**
   δ ([D₆]-DMSO) = 2.58 (s, 3H); 3.81 (s, 3H); 7.60-7.71 (m, 2H); 8.16-8.18 (m, 1H); 11.50 (br. s,1 H) ppm.
**I-32:**
   δ ([D₆]-DMSO) = 1.32 (t, 3H); 2.58 (s, 3H); 4.27-4.32 (q, 2H); 7.65-7.73 (m, 2H); 8.23-8.25 (m, 1H); 11.31 (br. s,1 H) ppm.
**I-33:**
   δ ([D₆]-DMSO) = 2.38 (s, 3H); 2.59 (s, 3H); 7.65-7.75 (m, 2H); 8.23-8.25 (m, 1H); 11.95 (br. s,1 H) ppm.
**I-34:**
   δ ([D₆]-DMSO) = 1.21 (t, 3H); 4.18 (q, 2H); 5.43 (s, 2H); 7.38-7.89 (m, 8H); 9.38 (br. s,1 H) ppm.
**I-35:**
   δ ([D₆]-DMSO) = 1.33-1.36 (t, 3H); 4.34-4.40 (q, 2H); 8.01-8.04 (m, 1H); 8.51-8.54 (m, 1H); 8.72 (m,1 H); 11.02 (br. s,1 H) ppm.
   Die Bestimmung der ¹H-NMR-Daten erfolgt mit einem Bruker Avance 400, mit Tetramethylsilan als Referenz (0.0 ppm) und den Lösungsmitteln CD₃CN oder [D₆]-DMSO. Die Charakterisierung der Signalaufspaltung erfolgt mit s = Singulett, br. s = breites Singulett, d = Dublett, t = Triplett, q = Quartett, m = Multiplett, dd = doppeltes Dublett.

### Anwendungsbeispiele

### Myzus-Test (MYZUPE Spritzbehandlung)

- Lösungsmittel:: 78,0 Gewichtsteile Aceton 1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 70 % bei einer Aufwandmenge von 500 g/ha:

Bsp. Nr. I-2, I-3, I-4, I-5, I-6, I-7, I-8, I-10, I-11, I-12, I-13, I-16, I-17, I-18, I-19, I-20, I-21, I-22, I-23,1-24,1-25,1-26,1-27,1-28,1-29,1-30,1-31,1-32,1-33

Bei diesem Test zeigen z. B. die folgenden Verbindungen der Herstellungsbeispiele eine Wirkung von ≥ 80 % bei einer Aufwandmenge von 100 g/ha:

Bsp. Nr. I-9

## Patentansprüche

1. Verfahren zur Bekämpfung von tierischen Schädlingen, **gekennzeichnet dadurch, dass** eine pestizid wirksame Menge mindestens einer Verbindung der Formel (I), 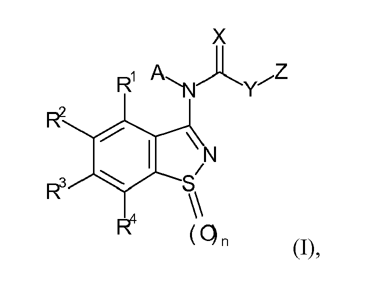 wobei
R¹, R², R³ und R⁴ unabhängig voneinander für
Wasserstoff, Halogen, Carbamoyl, Thiocarbamoyl, Nitro, Cyano, Hydroxy, SF₅ , C₁-C₆-Alkyl, C₂-C₆-Alkenyl, C₂-C₆-Alkinyl, C₃-C₆-Cycloalkyl, C₃-C₆-Cycloalkyl-C₁-C₄-Alkyl, C₁-C₄-Halogenalkyl, C₁-C₄-Halogenalkylthio, C₁-C₄-Alkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkylcarbonyl, C₁-C₆-Alkoxy, C₁-C₆-Alkoxycarbonyl, C₃-C₆-Alkenyloxy, C₃-C₆-Cycloalkyloxy, C₃-C₆-Cycloalkyloxy-C₁-C₄-Alkyl, C₁-C₄-Halogenalkoxy, C₁-C₄-Alkoxy-C₁-C₄-Alkyl, Aryloxy, Hetaryloxy, Aryl-C₁-C₄-Alkyloxy, Hetaryl-C₁-C₄-Alkyloxy, (C₁-C₄-Alkoxy)carbonyl, O-Acetyl, (C₁-C₄-Alkyl)amino, Di-(C₁-C₄-alkyl)amino, C₃-C₆-Trialkylsilyl, Aryl, Hetaryl, Aryl-C₁-C₆-Alkyl oder Hetaryl-C₁-C₆-Alkyl stehen,
wobei diese Reste unsubstituiert sein können oder einen, zwei oder mehrere Reste aus der Gruppe
Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl und Hetaryl tragen können;
A für Wasserstoff, (C₁-C₆-Alkyl)carbonyl, (C₁- C₆-Alkoxy)carbonyl, (C₁-C₆-Alkylthio)carbonyl, (C₁-C₆-Alkyl)thiocarbonyl, (C₁-C₆- Alkoxy)thiocarbonyl, (C₁-C₆-Alkylthio)thiocarbonyl, C₁-C₈-Alkyl, C₃-C₆-Cycloalkyl, C₂-C₆-Alkenyl, C₁-C₄-Alkoxy, C₁-C₄-Alkylthio, C₁-C₄-Alkylsulfinyl, C₁-C₄-Alkylsulfonyl, C₁-C₄-Halogenalkyl, Aryl, Hetaryl, C₁-C₆-Arylalkyl oder C₁-C₆-Hetarylalkyl steht,
wobei diese Reste unsubstituiert sein können oder einen, zwei oder mehrere Reste aus der Gruppe
Halogen, Cyano, C₁-C₆-Alkyl, C₃-C₈-Cycloalkyl, C₁-C₄-Alkoxy, C₁-C₄-Alkylthio, C₁-C₄-Alkylsulfinyl, C₁-C₄-Alkylsulfonyl, C₁-C₄-Halogenalkyl, C₁-C₄-Halogenalkoxy, C₁-C₄-Halogenalkylthio, (C₁-C₄-Alkyl)amino, Di-(C₁-C₄-alkyl)amino, Aryl und Hetaryl tragen können;
oder
A für den Rest 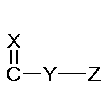 steht,
n für 0, 1 oder 2 steht,
X für O oder S steht,
Y für O, S, oder C(=O) steht,
Z für C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Aryl, Hetaryl, C₁-C₆-Arylalkyl, C₁-C₆-Hetarylalkyl, und für den Fall, dass Y für C(=O) steht, auch für C₁-C₈-Alkoxy, C₁-C₄-Haloalkoxy, Aryloxy oder Hetaryloxy steht,
wobei Aryl und Hetaryl gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, Cyano, Nitro, C₁-C₄-Alkoxy, C₁-C₄-Haloalkoxy substituiert sein können,
sowie Salze der Verbindungen der Formel (I)
auf tierische Schädlinge und/oder ihre Umgebung aufgebracht werden.

2. Verfahren zur Bekämpfung von tierischen Schädlingen gemäß Anspruch 1, **gekennzeichnet dadurch, dass** eine pestizid wirksame Menge mindestens einer Verbindung der Formel (I) mit mindestens einem Salz der Formel (IV) 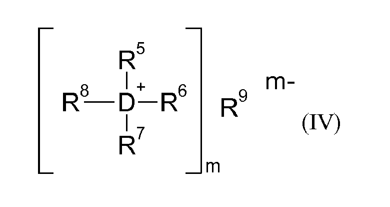 in welcher
D für Stickstoff oder Phosphor steht,
R⁵, R⁶, R⁷, and R⁸ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R⁹ für ein anorganisches oder organisches Anion steht,
auf tierische Schädlinge und/oder ihre Umgebung aufgebracht werden.

3. Verfahren gemäß Anspruch 1 oder 2, **dadurch gekennzeichnet, dass** mindestens ein Penetrationsförder der Formel (V)
R-O-(-AO)ᵥ₋R' (V)
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
v für Zahlen von 2 bis 30 steht,
auf tierische Schädlinge und/oder ihre Umgebung aufgebracht wird.

4. Verfahren gemäß Anspruch 1 bis 3, **gekennzeichnet dadurch, dass** Erntegut und/oder Pflanzenvermehrungsmaterial vor Befall durch tierische Schädlinge geschützt wird, wobei eine pestizid wirksame Menge mindestens einer Verbindung der Formel (I) gemäß Anspruch 1 auf das Erntegut und/oder das Pflanzenvermehrungsmaterial aufgebracht wird.

5. Verbindungen sowie Salze der Verbindungen der Formel (I) gemäß Anspruch 1,
wobei A nicht für Phenyl steht, falls n=2 ist,
und wobei R¹ verschieden von Wasserstoff ist, falls n=0 ist.

6. Verbindungen sowie Salze der Verbindungen der Formel (I) gemäß Anspruch 1 oder 5,
wobei
R¹ für Halogen, Nitro, C₁-C₃-Alkyl, C₁-C₃-Halogenalkyl, C₁-C₃-Alkylthio, C₁-C₃-Alkylsulfonyl, C₃-C₆-Alkenyloxy, C₁-C₃-Alkoxy, C₁-C₂-Halogenalkoxy, C₁-C₄-Alkoxy-C₁-C₄-Alkyl, Aryloxy, Hetaryloxy, C₁-C₂-Arylalkyloxy, C₁-C₂-Hetarylalkyloxy, C₁-C₄-Alkoxycarbonyl, O-Acetyl stehen,
wobei diese Reste unsubstituiert sein können oder einen, zwei oder mehrere Reste aus der Gruppe
Halogen, C₁-C₄-Alkoxy tragen können,
R², R³ und R⁴ für Wasserstoff stehen,
A Wasserstoff, (C₁-C₃-Alkyl)carbonyl, (C₁-C₃-Alkoxy)carbonyl, C₁-C₂-Alkyl, C₁-C₄-Alkoxy, C₁-C₄-Halogenalkyl, C₁-C₄-Halogenalkoxy, C₁-C₂-Arylalkyl oder C₁-C₂-Hetarylalkyl steht,
oder
A für den Rest 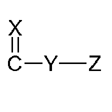 steht,
n für 0 oder 2 steht,
X für O oder S steht,
Y für O, S oder C(=O) steht,
Z für C₁-C₄-Alkyl, C₁-C₄-Haloalkyl, C₂-C₆-Haloalkinyl, Aryl und für den Fall, dass Y für C(=O) steht, auch für C₁-C₄-Alkoxy steht.

7. Verbindungen sowie Salze der Verbindungen der Formel (I) gemäß Anspruch 1 oder 5,
wobei
R¹, R², R³, R⁴ und A für Wasserstoff stehen,
n für 2 steht,
X für O oder S steht,
Y für O oder S steht,
Z für C₁-C₄-Alkyl, C₁-C₄-Haloalkyl, C₂-C₆-Haloalkinyl, Aryl steht.

8. Verfahren zur Herstellung von Verbindungen der Formel (I), **dadurch gekennzeichnet daß**
(A) 3-Amino-1,2-benzoisothiazole der Formle (II) 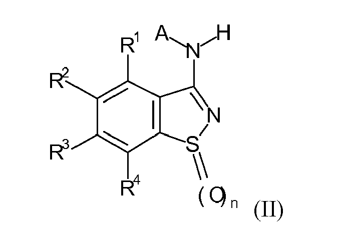 mit einem Säurederivat der allgemeinen Formel (III) 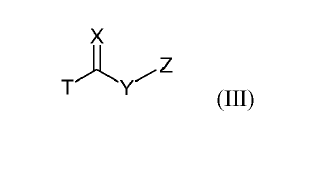 umgesetzt werden,
wobei R¹ bis R⁴, n, X, Y, Z und A die oben angegebenen Bedeutungen aufweisen
und T für Halogen und 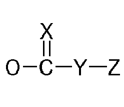 steht,
oder
(B) (Thio)carbamate und (Thio)oxamate der Formel (Ib) 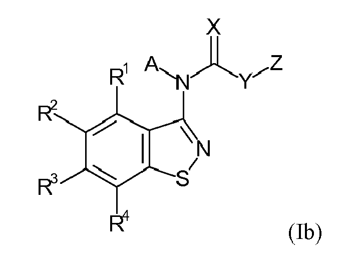 wobei R¹ bis R⁴, X, Y, Z und A die oben angegebenen Bedeutungen aufweisen,
mit einem Oxidationsmittel umsetzt,
oder
(C) (Thio)carbamate und (Thio)oxamate der Formel (Ic) 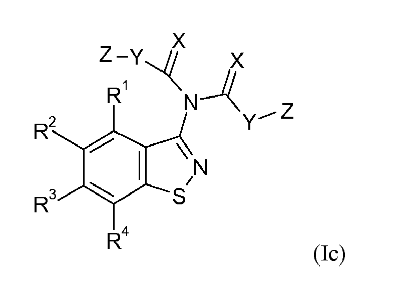 mit einem basischen oder sauren Spaltungsreagens unsetzt, wobei A Wasserstoff bedeutet und R¹ bis R⁴, X, Y und Z die oben angegebenen Bedeutungen aufweisen.
